# EUROPÄISCHE PATENTSCHRIFT

(11) **EP 0 722 075 B1**
(45) Veröffentlichungstag und Bekanntmachung des Hinweises auf die Patenterteilung: **01.09.1999**
(21) Anmeldenummer: 95108722.0
(22) Anmeldetag: 07.06.1995
(51) Int. Cl.: F28D 7/02

(54) **Hochleistungs-Kapillarwärmeaustauscher**
Heavy-duty capillary tube heat exchanger
Echangeur de chaleur à tubes capillaires de haute capacité

(30) Priorität: 10.01.1995 DE 19500421
(43) Veröffentlichungstag der Anmeldung: 17.07.1996
(73) Patentinhaber: hde Metallwerk GmbH, 58706 Menden (DE)
(72) Erfinder: Werner, Udo, Prof. Dr. Ing., D-45657 Recklinghausen (DE); Langer, Gert, Dr. Ing., D-58730 Fröndenberg (DE); Geissler, Stefan, Dipl.-Ing., D-44135 Dortmund (DE); Beykirch, Helmut, D-58710 Menden (DE); Schweitzer, Karl Heinz, D-59423 Unna (DE)
(74) Vertreter: Schulte, Jörg, Dipl.-Ing.

(56) Entgegenhaltungen:
- EP-A- 0 283 826
- WO-A-92/16807
- DE-B- 2 345 243
- H. TITZE : "Elemente des Apparatebaus" , 1963, SPRINGER VERLAG, BERLIN/GÖTTINGEN/HEIDELBERG

## Beschreibung

Die Erfindung betrifft einen Wärmeaustauscher für die thermische Konditionierung von Stoffgemischen oder für die Sterilisation von Flüssigkeiten, die mit Mikroorganismen kontaminiert sind oder sein können, mit mindestens einer Anschlußöffnung für ein Zentralrohr aufweisenden Eintritts- und Austrittsflansch und einem diese verbindenden Rohrbündel, das von einem Gehäuse mit Stutzen für die Zufuhr und Ableitung des Heizmediums gegen die Umgebung abgeschlossen ist. Die Erfindung betrifft außerdem ein Verfahren zur thermischen Konditionierung von Stoffgemischen oder für die Sterilisation von Flüssigkeiten.

Derartige Wärmeaustauscher werden unter anderem in der Lebensmitteltechnik, der pharmazeutischen Industrie und der Biotechnologie sowie in anderen Bereichen der Verfahrenstechnik dort eingesetzt, wo flüssige Medien in möglichst kurzer Zeit auf hohe Temperaturen erhitzt werden müssen. Dies erfolgt zur Sterilisation dieser Flüssigkeiten durch Abtöten unerwünschter Mikroorganismen und Keime.

Problematisch ist, daß durch die Hitzebehandlung auch hitzelabile Komponenten und Wertstoffe wie z. B. Vitamine und Proteine ebenfalls denaturiert werden, wobei für diesen negativen Effekt die Dauer der Hitzeeinwirkung von wesentlicher Bedeutung ist. Diese sogenannte Denaturierung tritt vor allem bei der sogenannten diskontinuierlichen Sterilisation mit ihrer meist langen Aufheiz-, Halte- und Abkühlzeit auf. Darüber hinaus muß bei der diskontinuierlichen Sterilisation nachteiligerweise auch die Verpackung miterhitzt und damit sterilisiert werden. Von daher wird die kontinuierliche Sterilisation bevorzugt, mit der kurze Verweilzeiten verwirklicht werden können. In der Lebensmitteltechnik ist insbesondere die Ultrahochtemperaturerhitzung von Milch bekannt.

Für diese bekannte kontinuierliche Sterilisation werden industriell im allgemeinen Plattenwärmetauscher eingesetzt. Diese bestehen aus übereinander geschichteten Platten mit speziellen, die Fließkanäle ausbildenden wellenförmigen Einprägungen. Diese Platten werden meist in größerer Zahl mittels Zuganker zwischen dickwandigen Gestellplatten zusammengepreßt und stützen sich, je nach Wellenmuster, an vielen Punkten gegenseitig ab. Der Abstand der Platten variiert zwischen 2,5 und 12 mm, so daß dementsprechend unterschiedlich große Strömungskanäle entstehen. Zwischen jeweils zwei Platten fließen abwechselnd das Wertprodukt und das Wärmeübertragungsmedium. Konstruktionsbedingt sind die Strömungswege des Wertproduktes in den einzelnen Kanälen von der Zulauf- bis zur Ablauföffnung bei sämtlichen Bauarten unabhängig von der Art der Überströmung der Platten, wobei Diagonalstrom- und Bogenstromführung zu unterscheiden sind, unterschiedlich lang. Daraus ergibt sich zwangsweise eine entsprechend breite Verweilzeitverteilung der zu sterilisierenden Medien in diesen bekannten Plattenwärmeaustauschern mit der Folge, daß ein bestimmter Anteil der hitzeempfindlichen Bestandteile, deren Aufenthaltszeit im Wärmeaustauscher über der mittleren Verweilzeit liegt, einer stärkeren Denaturierung unterliegt. Eine noch größere Verweilzeitverteilung tritt durch die an den Berührungspunkten benachbarter Platten auftretenden hydraulischen Grenzschichten bzw. Totgebiete auf, in denen die Strömungsgeschwindigkeit naturbedingt auf sehr kleine Werte absinkt. Eine Kapillarfilteranordnung ist aus der EP-A1 - 0 283 826 bekannt. Sie dient zur Sterilisation von Flüssigkeiten und arbeitet mit Kapillarfaserbündeln, die von Verteilungsräumen aus mit der Flüssigkeit versorgt werden. Eine genaue Zuweisung zu den einzelnen Kapillaren und eine gleichmäßige Erwärmung ist nicht gewährleistet.

Aus der allgemeinen Technik sind Röhrenwärmeaustauscher bekannt (DE-AS 23 45 243), die vorteilhaft große Durchströmungsquerschnitte und als parallele Rohre gleichlange Strömungswege aufweisen. Flüssigkeiten, die diese Röhren durchströmen, haben daher als Rohre eine ähnliche Verweilzeitverteilung. Nachteilig ist allerdings hier und auch bei dem Wärmetauscher nach der PCT-WO-92/16807, daß durch die entsprechend unterschiedliche Zuführung des Mediums vom zentralen Zulaufrohr aus gesehen trotz der nur vier Röhren wiederum stark ungleiche Strömungswege und damit unterschiedliche Verweilzeiten auftreten. Auch ansonsten sind diese bekannten Röhren-Wärmeaustauscher für die vorgesehene Sterilisation von Flüssigkeiten bzw. für die Konditionierung von Stoffgemischen ungeeignet, weil sie eine Kurzzeitsterilisation im Sekundenbereich nicht ermöglichen.

Der Erfindung liegt daher die Aufgabe zugrunde, einen Wärmetauscher und ein Verfahren zu schaffen, die auch für die Kurzzeitsterilisation geeignet sind und eine gleiche Verweilzeit und Sterilisationstemperatur gewährleisten.

Die Aufgabe wird erfindungsgemäß durch die Merkmale des Patentanspruchs 1, bzw. des Patentanspruchs 27 gelöst.

Durch dieses Wärmeaustauscherkonzept, bei dem das Medium, vor allem zu sterilisierendes Blut, durch mehrere gleichlange und über einen gleichen Querschnitt verfügende Rohre strömt und auch noch gleichmäßig auf die Rohre verteilt wird, wird eine Verweilzeitverteilung in sehr engen Grenzen gewährleistet. Das Medium, das durch die Rohre mit dem entsprechenden Strömungsquerschnitt hindurchgeführt wird, kann beispielsweise in zehntel Sekunden auf 140 °C erhitzt werden, so daß damit die notwendige Sterilisation unter gleichzeitiger weitestgehender Schonung der Vitamine und Proteine gewährleistet werden kann. Damit ist es überraschend möglich, eine Art Rohrbündel-Wärmeaustauscher auch in der Lebensmitteltechnik und verwandten Techniken, wie der Medizintechnik, einzusetzen und zwar mit der Folge, daß mit diesen Wärmeaustauschern gleiche Verweilzeiten und gleichmäßige Sterilisationstemperatur gewährleistet sind. Die Rohre weisen einen sehr engen Strömungsquerschnitt und eine dünne Wandstärke auf, so daß auf kurzem Wege eine gleichmäßige und schnelle Temperaturerhöhung für das Medium in den Rohren erreicht werden kann. Die zum Einsatz kommenden gleichlangen Kapillarrohre mit sehr engem Strömungsquerschnitt ermöglichen es dabei beispielsweise auch hochwertige Lebensmittel oder ähnliche Güter schonend und doch in der notwendigen Zeit und mit der notwendigen Temperatur zu sterilisieren, ohne daß die Gefahr besteht, daß die eigentliche Flüssigkeit dabei Schaden nimmt.

Nach einer zweckmäßigen Ausführung der Erfindung ist vorgesehen, daß die Rohre einen kreisförmigen oder ovalen Querschnitt aufweisen. Zwar können die Rohre vom Prinzip her eine beliebige Querschnittsform haben, doch sind die beschriebenen kreisförmigen oder ovalen Rohre gut und sicher zu entsprechenden Rohrbündeln zu verarbeiten und zwar auch dann, wenn sie die erfindungsgemäß geringe bzw. dünne Wandstärke aufweisen. Sie können darüber hinaus auch einen flachen bzw. rechteckförmigen Querschnitt aufweisen, wobei diese Form den Vorteil hat, daß bezogen auf das Flüssigkeitsvolumen, in den Kapillaren eine größere Wärmeübertragungsfläche vorhanden ist.

Um die Erhitzung des Mediums auf z. B. 140 °C innerhalb des notwendigen kurzen Zeitraumes zu realisieren, werden entsprechend kurze Rohre mit einem Innendurchmesser bzw. Kantenlängen von 0,5 bis 5 mm, vorzugsweise 1,0 bis 3,0 mm eingesetzt. Diese Rohre geben aufgrund des relativ geringen Querschnittes eine definierte Länge und eine definierte Strömung vor, so daß dementsprechend auch eine genaue Überwachung bzw. Einhaltung der vorgesehenen Temperaturwerte möglich ist. Dies gilt auch bezüglich der weiteren Ausbildung, nach der vorgesehen ist, daß die Rohre eine Wandstärke von 0,05 bis 1 mm, vorzugsweise 0,1 bis 0,3 mm aufweisen. Damit kann bei gleichzeitig genau definiertem Querschnitt eine gleichmäßige Erhitzung des Mediums in kürzester Zeit sichergestellt werden.

Auch bei geringsten Strömungsgeschwindigkeiten (laminarer Strömungsbereich) ist die gewünschte Erhitzung unter optimalen Bedingungen sichergestellt, da nach einer weiteren zweckmäßigen Ausbildung der Erfindung vorgesehen ist, daß die Rohre über den Querschnitt des Gehäuses gleichmäßig verteilt angeordnet und wendel- oder mäanderförmig gebogen oder gewickelt ausgebildet sind. Unabhängig vom Querschnitt dieser entsprechend wendel- oder mäanderförmig gebogenen bzw. gewickelten Rohre werden der Hauptströmung sogenannte Sekundärströmungen quer zur axialen Strömung überlagert, wodurch der Impuls- sowie der Wärmeaustausch durch vermehrte Konvektion deutlich gesteigert wird. Da über die Länge der Rohre gesehen immer wieder solche erzwungen Strömungsumlenkungen auftreten, kommt es zu der schon erwähnten deutlichen Verbesserung bzw. Beschleunigung des Wärmeaustauschers sowie einer Einengung der Verweilzeitverteilung. Vorteilhaft ist darüber hinaus, daß durch diese Anordnung und Ausbildung der Rohre eine deutliche Verkleinerung der Abmessungen der einzelnen Wärmeaustauscher bis in den Milliliterbereich möglich ist.

Ebenfalls zur Steigerung des Wärmeübergangs kann es zweckmäßig sein, die Innen- und/oder Außenwände der Rohre profiliert auszubilden. Durch diese Strömungshindernisse an den Innenwänden bzw. den Außenwänden wird die hydraulische Grenzschicht durch Strömungsablösungen (Verwirbelung) gestört und hierdurch der Impuls- und Wärmeaustausch quer zur Hauptströmungsrichtung deutlich verbessert.

Weiter vorne ist darauf hingewiesen worden, daß erfindungsgemäß sichergestellt ist, daß über Zuteilungskanäle die einzelnen Rohre jeweils gleichmäßig mit dem flüssigen Medium beaufschlagt sind. Dies wird nun insbesondere dadurch erreicht, daß die Zuteilungskanäle sternförmig von der zentralen, mit dem Zentralrohr verbundenen Bohrung auf die in gleichmäßigem Abstand kreisförmig um die Mittelachse angeordneten Rohre zulaufend ausgebildet sind. Werden beispielsweise zehn Rohre eingesetzt, so gehen von der zentralen Bohrung zehn gleichmäßig ausgebildete Zuteilungskanäle radial nach außen und sorgen somit dafür, daß die einzelnen Rohre alle über das Zentralrohr von vornherein gleichmäßig mit Medium beaufschlagt sind. Die Zuteilungskanäle sind gleich ausgebildet und auch gleich lang, so daß unabhängig von der Strömungsgeschwindigkeit eine Kurzzeitsterilisation gezielt und bei Einhaltung gleicher Verweilzeiten durchgeführt werden kann.

Variiert die Zuführung von zu sterilisierendem zu konditionierendem Medium, so kann es zweckmäßig sein, wenn den Zuteilungskanälen einzeln oder gruppenweise wirkende Regelorgane zugeordnet sind, so daß zeitweise einzelne der Zuteilungskanäle oder ganze Gruppen stillgelegt werden, wodurch durch die übrigen Zuteilungskanäle und Rohre Medium mit gleichmäßiger Strömungsgeschwindigkeit geführt wird. Durch Beaufschlagung verschiedener Kapillarrohre kann die Durchflußmenge variiert werden.

Einmal um die gleichmäßige Zuleitung des Mediums zu den Rohren zu erleichtern und zum anderen um ggf. auch entsprechende Regelorgane unterbringen zu können, ist vorgesehen, daß die Zuteilungskanäle im Eintrittsflansch bzw. Austrittsflansch integriert ausgebildet sind und daß der zugehörige Stirnflansch des Gehäuses eine durchgehend plane Fläche aufweist. Damit ist gleichzeitig eine metallische Abdichtung geschaffen, die vorteilhafterweise die gleichmäßige Zuführung des Mediums zu den Rohren durch die Zuteilungskanäle hindurch absichert, ohne daß es weiterer Absicherungsmaßnahmen bedarf.

Statt der schon erwähnten Regelorgane kann auch eine Veränderung der Anzahl der wirksamen Zuteilungskanäle dadurch erreicht werden, daß jeweils der die Zuteilungskanäle aufweisende Eintrittsflansch bzw. auch Austrittsflansch ausgewechselt wird. Dies ist problemlos möglich, da gemäß einer zweckmäßigen Ausbildung der Erfindung die beiden Stirnflansche über Stützrohre verbunden sind, die Verbindungsschrauben aufnehmen und daß die Stirnflansche und der Eintritts- bzw. der Austrittsflansch über getrennte Halteschrauben verbunden sind. Damit kann praktisch ohne Beeinflussung des übrigen Wärmeaustauschers lediglich der Eintritts- bzw. Austrittsflansch vom jeweiligen Stirnflansch gelöst werden, um gegen einen eine andere Zahl von Zuteilungskanälen aufweisenden Flansch ersetzt zu werden. Zweckmäßig können die beiden Stirnflansche mit dem Gehäuse durch Löten oder Schweißen direkt verbunden sein.

Die erfindungsgemäße Ausbildung des Wärmeaustauschers ermöglicht darüber hinaus eine modulare Parallelschaltung mehrerer derartiger Wärmeaustauscher oder aber eine Reihenschaltung, was dadurch ermöglicht ist, daß Eintritts- und Austrittsflansch mit den Gehäusen weiterer Wärmetauschermodule korrespondierend und koppelbar ausgebildet sind. Weiter hinten ist noch erläutert, daß dadurch auch die Möglichkeit gegeben ist, eine Kompletteinheit aus mehreren derartiger Wärmeaustauscher zu bilden, die vom Aufbau her alle gleich sind und die somit auch gleiche Leistungsdaten aufweisen. Die Reihenschaltung hat darüber hinaus den Vorteil, daß bei Bedarf auch die Verweilzeit dann gezielt erhöht werden kann, wenn dies aus irgendwelchen Gründen erforderlich ist, wobei einfach ein entsprechend bemessenes bzw. ausgebildetes Modul "angehängt" wird. Das Haltemodul kann auch andere geeignete Strömungsrrohre aufweisen, wobei die "Schnittstellen" entsprechend konstruktiv ausgebildet sein können.

Als Heiz- bzw. Kühlmedien können Dämpfe, Gase oder Flüssigkeiten verwendet werden, wobei die Zuleitungen sicher am Gehäuse befestigt werden können, das mit entsprechend tangential angeordneten Heizmediumstutzen ausgerüstet ist. So ist eine gleichmäßige Verteilung des Heizmediums im Innenraum des Gehäuses sichergestellt.

Um sowohl einen Gegenstrom- als auch einen Gleichstrombetrieb beispielsweise mit Wärmeüberträgerölen zu ermöglichen, ist erfindungsgemäß vorgesehen, daß eingangs- und ausgangsseitig zwei um 180° versetzte Heizmediumstutzen vorgesehen sind. Diese Heizmediumstutzen sind jeweils in der Nähe des Stirnflansches auf der Eintritts- und Austrittsseite angeordnet.

Um eine mechanische Verformung der dünnwandigen Rohre über die Länge des Gehäuses und auch um eine Verschiebung zueinander zu verhindern, wird die Lage der Rohre über im Abstand über die Länge des Gehäuses verteilt angeordnete Halterungen stabilisiert. Dabei sind die Halterungen so vorgesehen und ausgebildet, daß sie den Wärmefluß nur geringfügig behindern.

Im Falle der gewendelten Kapillarrohre sind die Halterungen als sternförmige Trägerelemente ausgebildet, bei der mäanderförmigen Rohrschlange dagegen als Distanzscheiben, wobei letztere dafür sorgen, daß die Rohre auf Abstand gehalten und gleichzeitig stabilisiert werden. Für flachovale Rohre ist eine ähnliche Halterung vorgesehen.

Bei Parallelschaltung mehrerer Wärmetauschermodule ist es von Vorteil, wenn diese sternförmig parallel geschaltet sind. Dadurch kann die Menge des zu sterilisierenden Mediums entsprechend gesteigert werden, ohne daß es aufwendigerer, weiterer Konstruktionen bedarf. Diese vorgesehene Anordnung hat darüber hinaus den Vorteil, daß ohne großen Aufwand auch einzelne der parallel geschalteten Wärmetauschermodule stillgelegt werden können, wenn die durchzusetzende Menge reduziert wird oder reduziert werden muß.

Weiter vorn ist darauf hingewiesen worden, daß neben der Parallelschaltung auch eine Reihenschaltung von baugleichen Wärmetauschermodulen möglich ist, wobei gemäß der Erfindung drei baugleiche Wärmetauschermodule eine Kompletteinheit mit Aufheiz-, Halte- und Kühlstufe bildend miteinander verbunden sind. Dies hat den großen Vorteil, daß ohne weitere Zwischenführung des zu sterilisierenden Mediums eine Komplettsterilisation von Normaltemperatur über die Aufheizung und die Kühlung wieder zurück zur Normaltemperatur erfolgen kann. Dabei ist über die gesamte Länge der Kompletteinheit jeweils im einzelnen Rohr eine gleichmäßige Führung des Mediums bei optimaler Beeinflussung sowohl bezüglich Aufheizung wie auch Kühlung gewährleistet und zwar unter Einhaltung einer engen und stets gleichen Verweilzeitverteilung.

Zur Vereinfachung dieser Kompletteinheit sieht die Erfindung ergänzend vor, daß die mittlere Haltestufe ohne Zwischenschaltung von Eintritts- bzw. Austrittsflansch direkt mit der Aufheiz- und der Kühlstufe verbunden ist, so daß nur endseitig der Aufheiz- bzw. Kühlstufe entsprechende Eintritts- und Austrittsflansche benötigt werden.

Zur Vermeidung von Anhaftung der Produktkomponenten an den Wänden der Rohre ist gemäß der Erfindung vorgesehen, daß die Zuleitungskanäle und/oder die Rohre eine Innenwandbeschichtung aufweisen, die aus einem hydro- oder lyophoben Material, vorzugsweise Paraffin bestehen. Auf diese Weise werden Anlagerungen verhindert, die zwangsweise zu negativen Grenzflächeneffekten führen würden, insbesondere aber auch dazu, daß die gewünschte gleichmäßige Erwärmung der Flüssigkeit bzw. der Stoffgemische nicht eintritt.

Denkbar ist es auch, daß die Zuteilungskanäle und/oder die Rohre insgesamt aus einem hydro- oder lyophoben Werkstoff bestehend ausgebildet sind, wobei dafür sowohl die Herstellung der Rohre aus Teflon, Polypropylen oder ähnlichem Material möglich ist.

Eine Optimierung der Verweilzeit ist dadurch zu erreichen, daß die Zuteilungskanäle mit Verbindungsstutzen ausgerüstet sind, die mit einem ein Trennmedium aufweisenden und über ein Zuteilungssystem verfügenden Behälter verbunden sind. Über das Zuteilungssystem wird aus dem Behälter in vorgegebenen zeitlichen Intervallen ein Trennmedium in den Flüssigkeitsstrom eingegeben, so daß sich Pfropfen bilden, die die einzelnen Flüssigkeitsteilmengen voneinander trennen. Durch diese Trennung der einzelnen Flüssigkeitpfropfen wird eine Rückvermischung vermieden und die Behandlung jeweils definierter Mengen während des Weges durch die Kapillarrohre gewährleistet. Diese vorteilhafte Begrenzung der Verweilzeitverteilung wird dadurch unterstützt, daß innerhalb jedes einzelnen Flüssigkeitspfropfens eine Zirkulationsströmung auftritt und die hierdurch innerhalb eines Pfropfens hervorgerufene Durchmischung der Flüssigkeit ebenfalls zur weiteren Einengung der Verweilzeitverteilung beiträgt.

Diese vorteilhafte Verhinderung einer Rückvermischung einerseits und einer Zirkulationsströmung andererseits wird insbesondere dadurch erreicht, daß der Behälter als Inertgas- oder Dampfbehälter ausgebildet ist, so daß die zwischengeschobenen Pfropfen Gasblasen sind, und zwar eben aus Inertgas bzw. aus Wasserdampf.

Um weitgehende Variationsmöglichkeiten zu haben, ist es von Vorteil, wenn das Zuteilungssystem alle, einzelne oder Gruppen von Zuteilungskanälen eine segmentierende Flüssigkeitsströmung erzielend ausgelegt ist. Das Zuteilungssystem weist hierzu getrennt zu betätigende Ventile auf oder ähnliche Regelteile, um auf diese Art und Weise den Zeitpunkt und auch die Menge der einregulierten Trennmedienmengen jeweils verändern bzw. ggf. auch verhindern zu können.

Zur thermischen Konditionierung von Stoffgemischen oder zur Sterilisation von Flüssigkeiten, die mit Mikroorganismen kontaminiert sind oder sein können, dient ein Verfahren, bei dem das indirekte Erhitzen der Stoffgemische bzw. Flüssigkeiten auf ganz besondere Art und Weise erfolgt, und zwar dadurch, daß der Stoffgemisch- oder Flüssigkeitsstrom in eine, genau definierte und ungefähr die gleiche Menge aufweisende Teilströme unterteilt und jeder Teilstrom mit gleicher Geschwindigkeit für einen vorgegebenen Zeitraum indirekt durch die Rohrwandung hindurch gleichmäßig und schnell erhitzt und in die einzelnen Teilströme im vorgegebenen Abstand ein sich mit diesem nicht mischendes Trennmedium eingegeben wird. Dieses Verfahren wird durch einen Wärmetauscher verwirklicht, der in den vorhergehenden Ansprüchen 1 bis 27 bzw. durch einzelne der dort beschriebenen Merkmale gekennzeichnet ist. Durch die Aufteilung des Flüssigkeitsstromes in möglichst kleine und dabei insbesondere bezüglich der Menge genau definierte Teilströme ist es möglich, diese vielen Teilströme in der Zeiteinheit gleichmäßig zu erwärmen, zu erhitzen und eine vorgegebene Zeit auf dieser Temperatur zu halten und auch ggf. nachfolgend wieder abzukühlen. Dabei ist eine Verweilzeitverteilung vorgegeben, die soweit eingeengt ist, daß die angestrebte gleichmäßige und wirksame Erwärmung des Flüssigkeitsstroms bzw. Stoffgemischstroms gewährleistet ist.
Die Einleitung der Trennmedium-Stopfen verbessert die Überwachung und Regelung der Aufheizung des Mediums.

Um die Verweilzeitverteilung weiter einengen zu können, kann es zweckmäßig sein, die Teilströme in miteinander verbundenen Modulen getrennt aufzuheizen, auf Temperatur zu halten und anschließend zu kühlen und dann erst wieder zusammenzuführen. Insbesondere der Abkühlung wegen kann eine solche Aufteilung in unterschiedliche Moduleinheiten zweckmäßig sein, weil dann dafür auch die notwendigen Gegebenheiten durch planmäßiges Abkühlen am besten zu verwirklichen sind.

Anlagerungen von einzelnen Produktkomponenten oder auch Produktgemischen werden wirksam verhindert, indem man die Zuteilungskanäle und/oder die Rohre mit einer Innenwandbeschichtung aus einem Grenzflächeneffekte unterbindenden Werkstoff, vorzugsweise aus Paraffin oder ähnlichen Verbindungen versieht. Diese Beschichtung beispielsweise in Form von Paraffin führt zur Erhaltung der Produktströme bzw. der Flüssigkeitsströmung, ohne daß die Gefahr besteht, daß diese über eine Anlagerung an der Innenwand beeinträchtigt wird.

Statt der Innenwandbeschichtung ist auch eine Ausbildung denkbar, bei der die Zuteilungskanäle und/oder die Rohre insgesamt aus einem hydro- bzw. lyophoben Werkstoff wie Teflon oder Polypropylen gefertigt werden.

Durch die gezielte, hintereinander folgende Einbringung von einem Trennmedium über ein geeignetes Zuteilungssystem in einzelne oder auch alle Rohre wird eine segmentierte Flüssigkeitsströmung erzielt, so daß die einzelnen Flüssigkeitssegmente durch die dann vorliegende Pfropfenströmung mit äußerst definierter Verweilzeit das System passieren. Gemäß Verfahren ist vorgesehen, daß in die Teilströme im vorgegebenen Abstand ein sich mit diesen mischendes Trennmedium eingegeben wird, wobei als Trennmedium zweckmäßigerweise Inertgas- oder Dampfblasen verwendet werden. Das Trennmedium, vorzugsweise in Form von Inertgas- oder Dampfblasen wird dabei in einen, mehrere oder in Gruppen von Teilströmen eingeschleust. Die äußerst genau definierte Verweilzeit durch die Pfropfenbildung beruht darauf, daß durch die zwischen den Flüssigkeitspfropfen befindlichen Gasblasen eine axiale Rückvermischung zwischen mehreren Flüssigkeitspfropfen unterbunden und auf diese Weise eine sehr schmale Verweilzeitverteilung gewährleistet wird. Diese vorteilhafte Begrenzung der Verweilzeitverteilung wird dadurch unterstützt, daß nun innerhalb jedes einzelnen Flüssigkeitspfropfens eine Zirkulationsströmung auftritt und die hierdurch innerhalb eines Pfropfens hervorgerufene Durchmischung der Flüssigkeit entsprechend sich positiv
aufweist.

Die Erfindung zeichnet sich insbesondere dadurch aus, daß ein Wärmeaustauscher geschaffen ist, der sowohl für die kontinuierliche Kurzzeitsterilisation wie auch für die Konditionierung von Stoffgemischen durch Hitzeeinwirkung unter optimal weitgehender Schonung hitzeempfindlicher Wertstoffe eingesetzt werden kann, wobei eine konstante Wärmezufuhr, sehr kurze Verweilzeiten und ein enges Verweilzeitspektrum eingehalten werden. Das Medium strömt durch gleichlange Rohre, die hier als Kapillarrohre wegen ihres engen Strömungsquerschnittes bezeichnet werden, wobei vor Eintritt in die Kapillarrohre durch gleichlange und gleichausgebildete Zuteilungskanäle vom zentralen Zulaufrohr her eine frühzeitige und absolut definierte Aufteilung des Mediums auf die Kapillarrohre erfolgt. Demzufolge hält sich die Verweilzeitverteilung in sehr engen Grenzen. Die Kapillarrohre selbst können beliebige Querschnittsformen besitzen, zweckmäßigerweise werden Rohre mit kreisförmigen oder flachem Querschnitt verwendet. Für die Wärmeerzeugung können beliebige Medien wie Dampf, Flüssigkeiten, elektrische Heizungen u. a. eingesetzt werden, ebenso auch für die Kühlung. Durch die besondere Art der Kapillarrohre ist eine optimale Wärmeübertragung gesichert, die noch durch Gegenstromführung, durch Schikanen oder Querströmung verbessert werden kann. Vor allem aber können die Kapillarrohre relativ einfach ersetzt werden, wobei das Gehäuse mit den Einzelteilen und den Dichtungen mehrfach Verwendung finden kann. Alternativ ist eine Version vorgesehen, bei der das Kapillarrohrbündel mit den Stirnflanschen direkt mit dem Gehäuse verschweißt oder verlötet ist. Vorteile sind die extrem schnelle Aufheizung und Abkühlung insbesondere bei den wendel- oder mäanderförmig gebogenen bzw. gewickelten Ausführungsformen. Auf das enge Verweilzeitspektrum ist schon hingewiesen worden. Zu erwähnen ist als weiterer Vorteil vor allem aber auch, daß kaum eine Gefahr der Verstopfung bei den Kapillarrohren mit dem runden und rechteckförmigen Querschnitt besteht, da Strömungseinbauten nicht vorhanden sind. Bei Verwendung entsprechender Materialien wie beispielsweise Edelstahl ist das Sterilisieren der gesamten Wärmeaustauscherausrüstung einfach möglich, wobei durch das Fehlen der Strömungseinbauten auch eine bessere Reinigung möglich ist. Aufgrund der immer gleichen Querschnitte, Mengen und Strömung ist eine einfache und absolut definierte Maßstabübertragung möglich, d. h. bei gleicher Bauform lassen sich beliebig viele, gleiche Module zusammenschalten, um größere Durchsätze zu erreichen. Die Flüssigkeiten durchlaufen überall die Rohre unter gleichen Bedingungen. Durch eine gezielte Einbringung von Inertgas- oder Dampfblasen über das Zuteilungssystem wird eine äußerst definierte Verweilzeit der einzelnen Flüssigkeitspfropfen bzw. der Flüssigkeitsströme gewährleistet. Nachteilige Grenzflächeneffekte können dann nicht auftreten, wenn die Rohre bzw. Zuteilungskanäle entweder mit hydro- oder lyophoben Material beschichtet sind oder aus entsprechendem Werkstoff hergestellt sind.

Weitere Vorteile des Erfindungsgegenstandes ergeben sich aus der nachfolgenden Beschreibung der zugehörigen Zeichnungen, in der bevorzugte Ausführungsbeispiele mit den dazu notwendigen Einzelheiten und Einzelteilen dargestellt sind. Es zeigen:
- Fig. 1: einen Wärmeaustauscher im Längsschnitt mit wendel-bzw. mäanderförmig gebogenen Rohren,
- Fig. 2: und
- Fig. 3: einen teilweisen Längsschnitt und einen teilweisen Querschnitt durch einen Wärmeaustauscher mit flachoval ausgebildeten Rohren,
- Fig. 4: einen Wärmeaustauscher im Querschnitt mit die Rohre stabilisierenden Halterungen,
- Fig. 5: eine Draufsicht auf die Stirnflanschseite eines Eintrittsflansches,
- Fig. 6: eine Kompletteinheit, bestehend aus mehreren Wärmeaustauschern,
- Fig. 7: ein flachovales Rohr mit Profilierung,
- Fig. 8: eine vergrößerte Darstellung des Rohres mit Profilierung,
- Fig. 9: einen Querschnitt durch das Rohr gemäß Fig. 7 und Fig. 8,
- Fig. 10: einen Längsschnitt durch ein Rohr mit durch Einspeisen von einem Trennmedium gebildeten Pfropfen,
- Fig. 11: einen Querschnitt durch ein Rohr mit Innenwandbeschichtung,
- Fig. 12: einen Längsschnitt durch ein Rohr mit Zuteilungssystemstutzen,
- Fig. 13: den oberen Teil eines Wärmetauschers mit Zuteilungssystem und
- Fig. 14: eine Draufsicht auf den Eintritts- oder Stirnflansch mit Teilen des Zuteilungssystems.

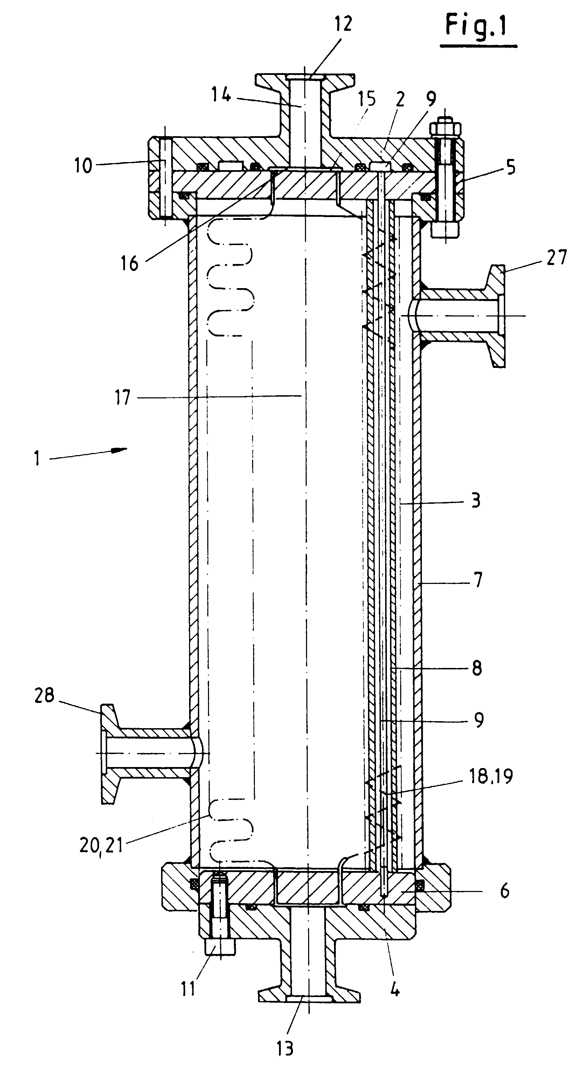

Fig. 1 zeigt einen Wärmeaustauscher 1 gemäß der Erfindung im prinzipiellen Aufbau, wobei die Ausbildung des Rohrbündels 3 zwischen Eintrittsflansch 2 und Austrittsflansch 4 in zwei Varianten wiedergegeben ist. Das Rohrbündel 3 ist jeweils endseitig durch Stirnflansche 5, 6 begrenzt und seitlich von einem rohrförmigen Gehäuse 7 umgeben. Jedem Einzelteil des Rohrbündels 3 ist ein Stützrohr 8 zugeordnet, das eine Verbindungsschraube 9 aufnimmt, so daß durch Anziehen der Verbindungsschraube 9 die beiden Stirnflansche 5, 6 gegeneinander die Stützrohre 8 und das gesamte Rohrbündel 3 festlegend gegeneinander verspannt werden können.

Außerdem sind die Stirnflansche 5, 6 mit dem Eintrittflansch 2 bzw. dem Austrittsflansch 4 über Halteschrauben 10, 11 getrennt verbunden, so daß der Eintrittsflansch 2 und der Austrittsflansch 4 getrennt von den jeweiligen Stirnflanschen 5, 6 lösbar sind.

Die zu behandelnde Flüssigkeit bzw. das Medium wird durch das obere Zentralrohr 12 und die mittige Bohrung 14 dem Wärmetauscher 1 zugeführt und über Zuteilungskanäle 15, 16 von vornherein gleichmäßig auf die einzelnen Rohre 18, 20 des Rohrbündels 3 verteilt. Die Rohre in Form der Wendel 19 oder der mäanderförmigen Rohrschlange 21 sind um die Mittelachse 17 gleichmäßig verteilt angeordnet, so daß über die Zuteilungskanäle 15, 16, die, wie weiter hinten noch erläutert wird, sternförmig verlaufen, alle Rohre 18, 20 gleichmäßig und gleichzeitig mit Medium beaufschlagt werden können.

Das Medium wird dann am unteren Ende über den Stirnflansch und die darin vorgesehenen Bohrungen bzw. Halterungen für die Rohre 18, 20 abgeführt und über die auch dort angeordneten Zuteilungskanäle wieder dem Zentralrohr 13 zugeführt und dann aus dem Wärmetauscher 1 abgeleitet.

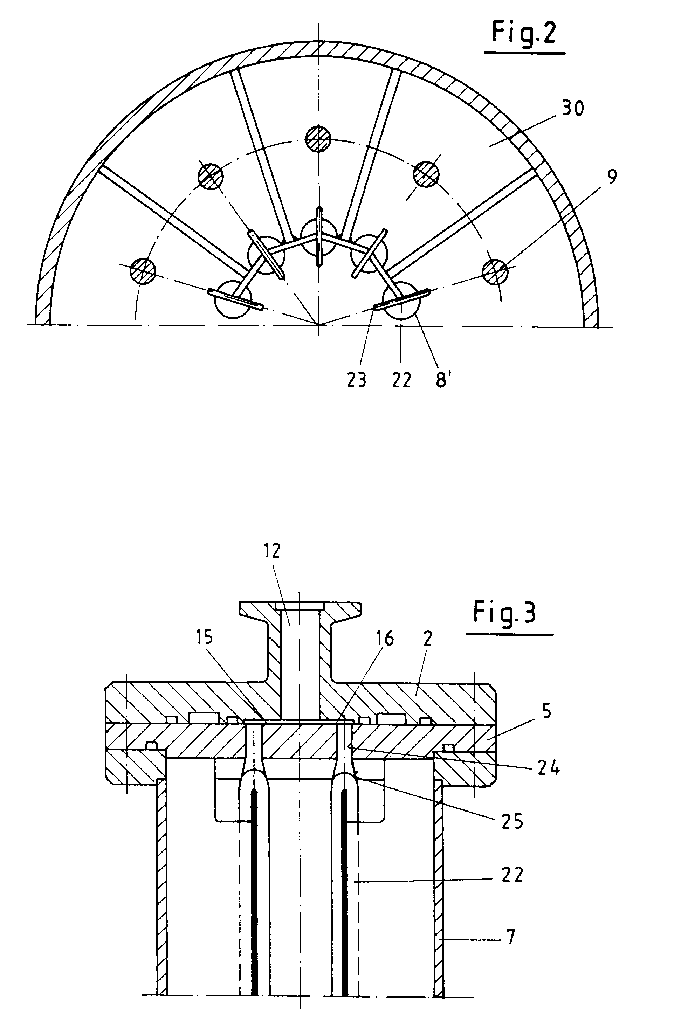

Statt der aus Fig. 1 ersichtlichen Ausführung der Rohre 18, 20 in Form der Wendel 19 bzw. der mäanderförmigen Rohrschlange 21 ist auch eine Verwendung in Form flachovaler Rohre 22 gemäß Fig. 2 und Fig. 3 denkbar, wobei sie mit oder ohne Profilierung 23 eingesetzt werden können. Bei Fig. 3 handelt es sich im oberen Teil um eine vergrößerte Wiedergabe der oberen Flansches 2 und 5 mit den entsprechenden Zuteilungskanälen 15 und 16. Die Profilierung 23 kann auf der Innenwand 24 oder der Außenwand 25 oder auch auf beiden Wänden vorgesehen werden.

Als Heiz- bzw. Kühlmedien können Dämpfe, Gase oder Flüssigkeiten wie Wärmeübertrageröl oder andere flüssige Medien eingesetzt werden. Dieses Heizmedium wird über die im Bereich des Flansches 2, 5 angeordneten Heizmediumstutzen 27 in das Gehäuse 7 hineingeleitet und über die Heizmedienstutzen 28 am unteren Flansch 4, 6 wieder abgeleitet. Diese Heizmedien- stutzen 27, 28 auf der Mantelseite des Wärmeaustauschers 1 sind bevorzugt tangential angeordnet, um eine gleichmäßige Verteilung des Heizmediums sicherzustellen. Es werden je zwei Stutzen um 180° versetzt in der Nähe des Stirnflansches 5 und auch des Stirnflansches 6 angebracht, um sowohl einen Gegenstrom- als auch einen Gleichstrombetrieb zu ermöglichen.

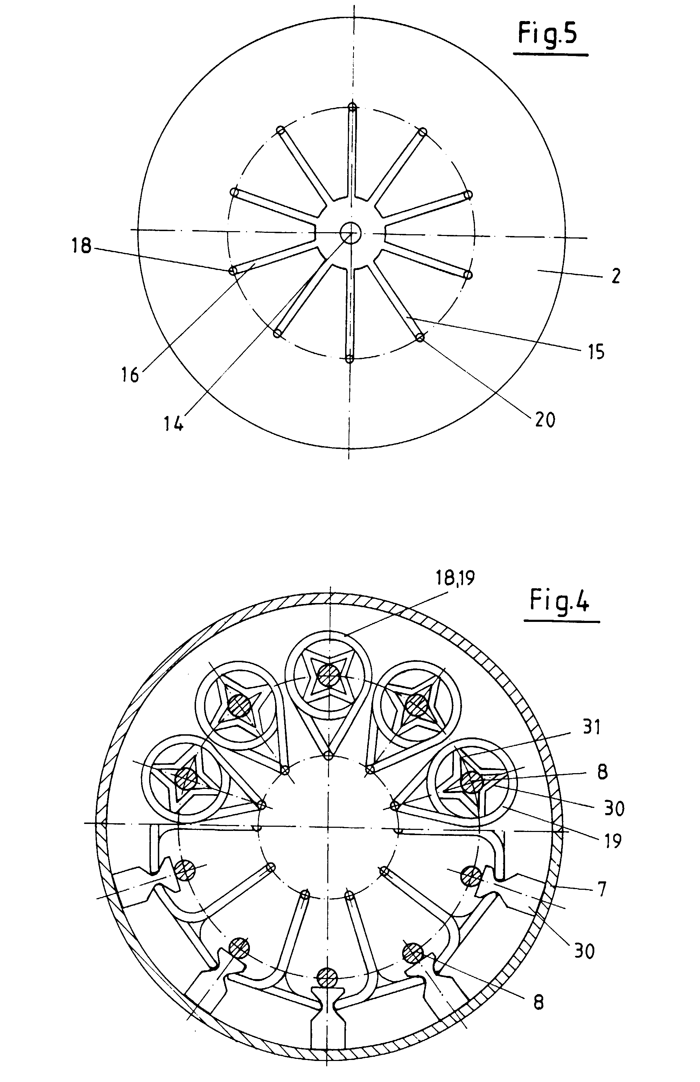

Um eine mechanische Verformung der Rohre 18, 20 bzw. eine Verschiebung der Rohre zueinander zu verhindern, werden die Rohrbündel bzw. wird das Rohrbündel 3 in sinnvollen Abständen mit Halterungen 30 versehen. Für diese Halterungen 30 sind unterschiedliche Ausführungsformen, wie in Fig. 2 und 4 angedeutet, denkbar. Im Falle der Wendel 19 werden die Rohre 18, 20 gemäß Fig. 4 auf z. B. sternförmigen Trägerelementen 31 aufgewickelt. Das Rohrbündel mit den mäanderförmig gebogenen Rohren 18, 20 wird durch mehrere, über die Lauflänge verteilte Distanzscheiben 32 gemäß Fig. 2 auf Abstand gehalten und mechanisch stabilisiert. Für die flachovalen Rohre 22 ist eine ähnliche Halterung 30 vorgesehen.

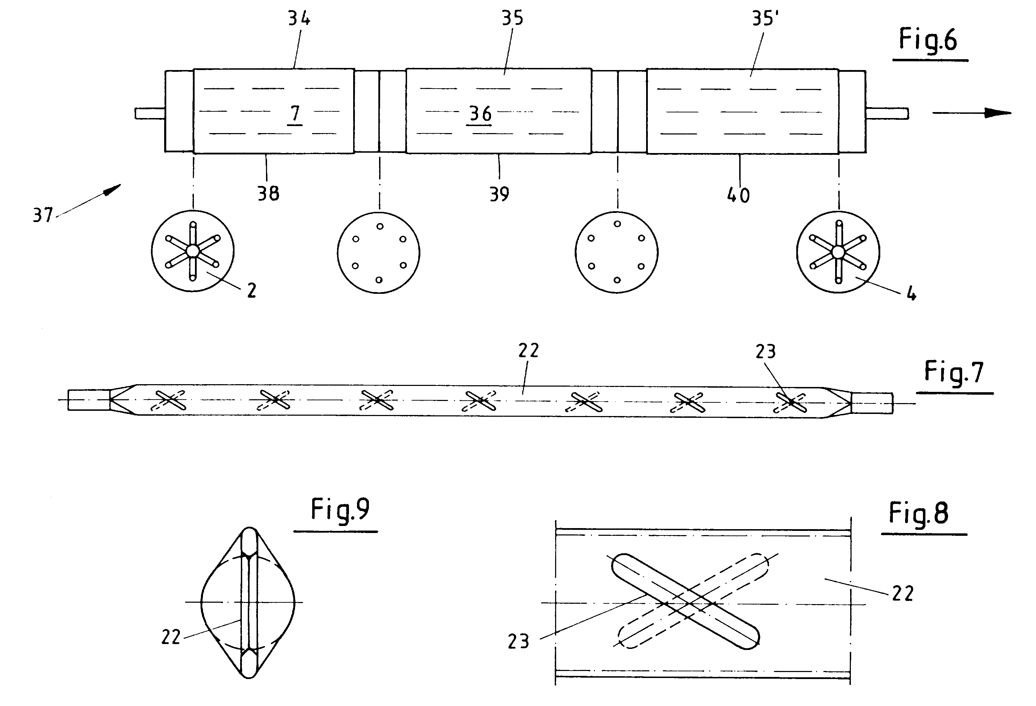

Fig. 5 zeigt eine Draufsicht auf die dem Stirnflansch 5 bzw. 6 zugewandte Seite des Eintrittsflansches 2 bzw. des Austrittsflansches 4. Genauer gesagt, handelt es sich hier um den Austrittsflansch 4, weil die die Köpfe der Verbindungsschrauben 9 aufweisenden Ausnehmungen bzw. eine entsprechend rillenförmige Nut bei dem aus Fig. 5 ersichtlichen Flansch nicht vorhanden ist. Deutlich ist aber, wie auch bei dem jeweiligen Eintrittsflansch 4 zu erkennen, daß beim Austrittsflansch 4 von der mittigen Bohrung 14 sternförmig ausgehende Zuteilungskanäle 15, 16 vorgesehen sind, die bis zu den Rohren 19, 20 reichen bzw. den in diese Kanäle übergehenden Bohrungen.

Bei der aus Fig. 5 ersichtlichen Ausführung sind zehn solcher Zuteilungskanäle 15, 16 entsprechend der Zahl der Bohrungen 18, 20 vorgesehen. Bei weniger derartigen Bohrungen 18, 20 reduziert sich auch die Zahl der Zuteilungskanäle bzw. dann, wenn einige der Zuteilungskanäle stillgelegt werden sollen, wird einfach ein Austrittsflansch 4 bzw. Eintrittsflansch 23 verwendet, der entsprechend über weniger Zuteilungskanäle 15, 16 verfügt.

Weiter vorn ist bereits darauf hingewiesen worden, daß es ein wesentlicher Vorteil dieses Wärmeaustauscherkonzeptes ist, daß durch modulare Parallelschaltung bei überall gleichlangen Zuteilkanälen 15, 16 quasi beliebig große Wärmeaustauscherflächen für die Behandlung entsprechend großer Volumendurchsätze realisiert werden können. Dabei ist sichergestellt, daß in sämtlichen Rohren 18, 20 dieselben Strömungs- und Wärmungsaustauschbedingungen herrschen und damit stets dieselbe enge Verweilzeitverteilung resultiert. Die Auslegung der erforderlichen Apparategröße bzw. der benötigten Wärmeaustauscherfläche gestaltet sich bei dieser Wärmeaustauscherkonzeption unter Gewährleistung höchster Ansprüche an Sicherheit und Genauigkeit problemlos.

Mit dem vorgeschlagenen Wärmeaustauscherkonzept läßt sich vorteilhafterweise sowohl das kurzzeitige und mit definierten Verweilzeiten ablaufende Aufheizen als auch Abkühlen von flüssigen Medien durchführen. Gemäß Fig. 6 sind mehrere Wärmetauschermodule 34, 35, 35' hintereinander geschaltet, um in dieser günstigen Form das flüssige Medium zu behandeln. Dabei kann die Kompletteinheit 37 in einem gemeinsamen Gehäuse 36 untergebracht sein oder aber in entsprechend zusammengeschalteten Teilgehäusen. Damit ist es möglich, in günstiger Form die flüssigen Medien zunächst in der Aufheizstufe 38 aufzuheizen, anschließend über definierte Zeitintervalle auf einer bestimmten Temperatur in der Haltestufe 39 zu halten und in einer dritten, nämlich der Kühlstufe 40 wieder definiert abzukühlen. Hiermit kann z. B. bei der Fermentation in Labor- und Technikumsanlagen jede Flüssigkeit in sehr kleinen Volumina mit sehr kleinen Volumenströmen z. B. von etwa 0,1 bis 10 l/h je nach Anforderung sterilisiert werden. Diese Aufgabe ist vorteilhafterweiser mit der aus Fig. 6 ersichtlichen Kompletteinheit 37 und damit in Form eines kontinuierlich betriebenen, kompakten Hochleistungskapillar-Wärmeaustauschers zu lösen.

Bei der aus Fig. 6 ersichtlichen Ausführung ist zwischen der Aufheizstufe 38 und der Haltestufe 39 sowie der Haltestufe 39 und der Kühlstufe 40 auf die Anordnung von Eintrittsflanschen 2 und Austrittsflanschen 4 verzichtet worden. Diese sind jeweils nur im Endbereich der Aufheizstufe 38 bzw. der Kühlstufe 40 vorgesehen. Im Zwischenbereich findet ein direkter Übergang von Rohr zu Rohr statt. Die entsprechenden Querschnitte unterhalb der Fig. 6 verdeutlichen dieses.

Fig. 7 zeigt ein flachovales Rohr 22 in Seitenansicht mit einer entsprechenden Profilierung 23. Ein Ausschnitt ist in Fig. 8 vergrößert wiedergegeben und Fig. 9 zeigt einen Querschnitt mit dem entsprechend aus einem Rundrohr erzeugten flachovalen Rohr 22.

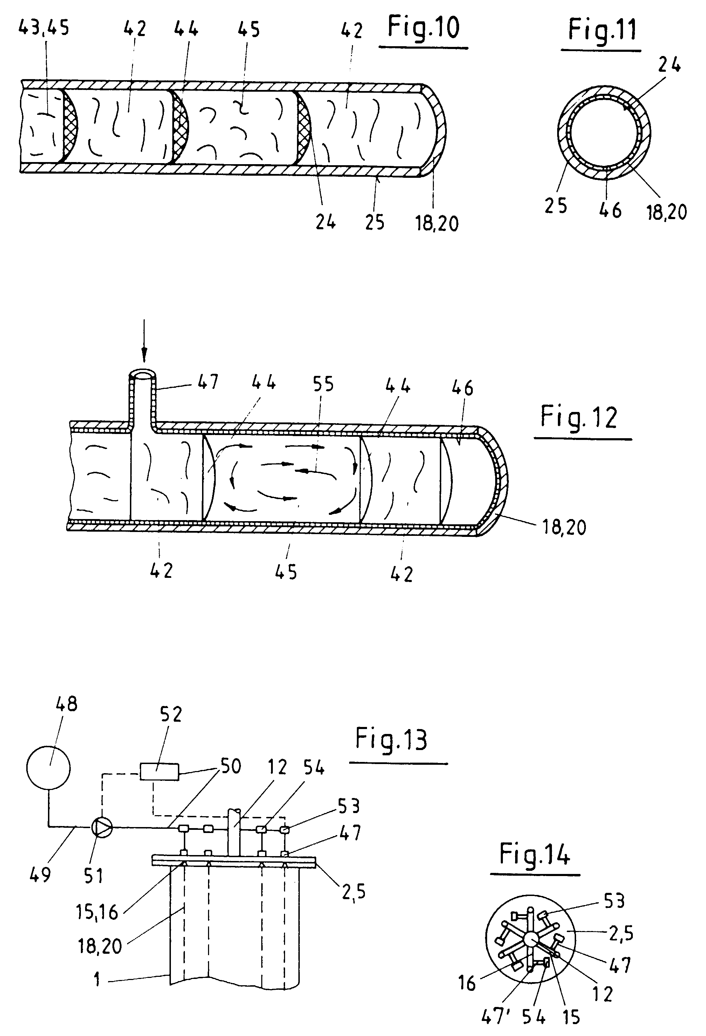

Fig. 10 zeigt einen Längsschnitt durch ein Rohr 18 bzw. 20, wobei hier gleichzeitig verdeutlicht ist, daß in die Flüssigkeit 43 jeweils im Takt ein Trennmedium 42 eingegeben ist, so daß über die sich dadurch einstellende Trennschicht 44 einzelne Flüssigkeitspfropfen gebildet werden, die besonders vorteilhaft gezielt zu erwärmen bzw. zu erhitzen sind. Das Trennmedium 42 kann dabei sehr genau getaktet eingegeben werden, so daß sich jeweils gleich große Flüssigkeitspfropfen 45 einstellen.

Fig. 11 zeigt einen Querschnitt eines entsprechenden Rohres 18, 20, wobei hier zur Vermeidung von Grenzflächeneffekten durch spezifische Anlagerung von Produktkomponenten o.ä. eine Innenwandbeschichtung 46 vorgesehen ist. Diese Innenwandbeschichtung 46 besteht hier beispielsweise aus Paraffin, wobei dafür Sorge zu tragen ist, daß die Beschichtung rundherum gleichmäßig ist. Hierbei ist weiter die Möglichkeit gegeben, auch das gesamte Rohr 18 bzw. 20 aus einem hydro- bzw. lyophoben Material herzustellen, also beispielsweise aus Teflon oder Polypropylen, um die beschriebenen negativen Effekte zu vermeiden.

Bei Fig. 12 ist die Darstellung nach Fig. 10 dahingehend ergänzt, daß hier der Längsschnitt mit einer entsprechenden Innenwandbeschichtung 46 wiedergegeben ist. Darüber hinaus ist ein Verbindungsstutzen 47 wiedergegeben, der an das hier nicht mehr dargestellte Zuteilungssystem 50 bzw. Leitungssystem 49 anschließt. Dabei werden über diesen Verbindungsstutzen 47 in vorgegebenen Takten Inertgas- oder auch Dampfblasen eingeführt, so daß sich die weiter oben beschriebenen Pfropfen aus Trennmedium 42 bzw. aus Inertblasen einstellen.

Ergänzend ist in Fig. 1 dargestellt, daß innerhalb der so gebildeten einzelnen Flüssigkeitspfropfen 45 eine Zwischenströmung 55 hervorgerufen wird, die zu einer vorteilhaften Durchmischung der Flüssigkeit und damit zur gleichmäßigen Beeinflussung und Erwärmung dieses einzelnen Flüssigkeitspropfen beiträgt. Fig. 2 verdeutlicht ergänzend auch, daß die Abmessungen der Flüssigkeitspfropfen 45 und der Gasblasen bzw. Trennmediumsblasen 42 nicht die gleichen Abmessungen aufweisen müssen, sondern daß diese vielmehr den jeweiligen Gegebenheiten weitreichend verändert werden können.

Die Figuren 13 und 14 zeigen einen Wärmetauscher 1, der zumindest auf der Seite des Eintrittsflansches 2 und/oder auch des Austrittsflansches 4 mit einem Zuteilungssystem 50 versehen ist. Dieses Zuteilungssystem 50 sorgt dafür, daß optimale Inertgasblasen bzw. Trennmediumsblasen 42 in die Flüssigkeit 43 bzw. in den Flüssigkeitsstrom eingeschleust werden, um die aus Fig. 10 und Fig. 12 ersichtliche Pfropfenbildung zu begünstigen. Das dazu benötigte Gas wird im Behälter 48 vorgehalten und über das Leitungssystem 49 dem jeweiligen Verbindungsstutzen 47 zugeführt. Im Bereich des Zuteilungssystem 50 bzw. in diesem ist eine Pumpe 51 angeordnet, die mit dem Steuerungsteil 52 zusammenarbeitet und dafür sorgt, daß über die Zwischenventile 53, 54 gleiche Mengen oder im vorgegebenen Takt entsprechende Mengen an Inertgas bzw. Dampf ins System einströmen, um die aus Fig. 10 und Fig. 12 ersichtliche Pfropfenbildung zu erreichen.

Über das Steuerungsteil 52 sowie die den einzelnen Verbindungsstutzen zugeordneten Zwischenventile 53, 54 ist es möglich, jeweils einzelne Rohre, alle Rohre oder aber auch Gruppen von Rohren 18, 20 bzw. Zuteilungskanäle 15, 16 mit dem Zuteilungssystem 50 zu verbinden, um entsprechende Blasen aus Trennmedium 42 zu erzeugen.

## Patentansprüche

1. Wärmetauscher (1) für die thermische Konditionierung von Stoffgemischen oder für die Sterilisation von Flüssigkeiten, die mit Mikroorganismen kontaminiert sind oder sein können, mit je einem mindestens eine Anschlußöffnung für ein Zentralrohr (12, 13) aufweisenden Ein- und Austrittsflansch (2, 4) und einem diese verbindenden Rohrbündel (3), das von einem Gehäuse (7) mit Stutzen (27, 28) für die Zufuhr und Ableitung des Heizmediums gegen die Umgebung abgeschlossen ist, wobei das Rohrbündel (3) mehrere gleichlange und über einen gleichen Querschnitt verfügende Rohre (18, 20) aufweist, jedes der Rohre (18. 20) über den gleichen Querschnitt und die gleiche Länge aufweisende separate Zuteilungskanäle (15, 16) mit dem jeweiligen Zentralrohr (12, 13) in Verbindung stehen und die Rohre als Kapillarrohre ausgebildet sind, deren definierter Querschnitt und Wandstärke eine gleichmäßige und schnelle Temperaturerhöhung ermöglichen.

2. Wärmeaustauscher nach Anspruch 1,
**dadurch gekennzeichnet,**
daß die Rohre (18, 20) einen kreisförmigen oder ovalen Querschnitt aufweisen.

3. Wärmeaustauscher nach Anspruch 1,
**dadurch gekennzeichnet,**
daß die Rohre (18, 20) einen rechteckförmigen Querschnitt aufweisen.

4. Wärmeaustauscher nach einem der vorhergehenden Ansprüche
**dadurch gekennzeichnet,**
daß die Rohre (18, 20) einen Innendurchmesser bzw. eine Kantenlänge von 0,5 bis 5 mm, vorzugsweise 1,0 bis 3,0 mm aufweisen.

5. Wärmeaustauscher nach einem der vorhergehenden Ansprüche,
**dadurch gekennzeichnet,**
daß die Rohre (18, 20) eine Wandstärke von 0,05 bis 1 mm, vorzugsweise 0,1 bis 0,3 mm aufweisen.

6. Wärmeaustauscher nach Anspruch 1,
**dadurch gekennzeichnet,**
daß die Rohre (18, 20) über den Querschnitt des Gehäuses (7) gleichmäßig verteilt angeordnet und wendel- oder mäanderförmig gebogen oder gewickelt ausgebildet sind.

7. Wärmeaustauscher nach Anspruch 1,
**dadurch gekennzeichnet,**
daß die Innen- und/oder Außenwände (24, 25) der Rohre (18, 20) profiliert sind.

8. Wärmeaustauscher nach Anspruch 1,
**dadurch gekennzeichnet,**
daß die Zuteilungskanäle (15, 16) sternförmig von der zentralen, mit dem Zentralrohr (12) verbundenen Bohrung (14) auf die in gleichmäßigem Abstand kreisförmig um die Mittelachse (17) angeordneten Rohre (18, 20) zulaufend ausgebildet sind.

9. Wärmeaustauscher nach einem der vorhergehenden Ansprüche,
**dadurch gekennzeichnet,**
daß den Zuteilungskanälen (15, 16) einzeln oder gruppenweise wirkende Regelorgane zur Variation der Durchflußmenge durch Beaufschlagung verschiedener als Kapillarrohre ausgebildete Rohre (18, 20) zugeordnet sind.

10. Wärmeaustauscher nach einem der vorhergehenden Ansprüche,
**dadurch gekennzeichnet,**
daß die Zuteilungskanäle (15, 16) im Eintrittsflansch (2) bzw. Austrittsflansch (4) integriert ausgebildet sind und daß ein zugehöriger Stirnflansch (5, 6) des Gehäuses (7) eine durchgehend plane Fläche aufweist.

11. Wärmeaustauscher nach Anspruch 10,
**dadurch gekennzeichnet,**
daß die beiden Stirnflansche (5, 6) über Stützrohre (8) verbunden sind, die Verbindungsschrauben (9) aufnehmen und daß die Stirnflansche (5, 6) und der Eintritts-bzw. der Austrittsflansch (2, 4) über getrennte Halteschrauben (10, 11) verbunden sind.

12. Wärmeaustauscher nach einem der Ansprüche 10 oder 11,
**dadurch gekennzeichnet,**
daß die beiden Stirnflansche (5, 6) mit dem Gehäuse (7) durch Löten oder Schweißen direkt verbunden sind.

13. Wärmeaustauscher nach einem der vorhergehenden Ansprüche,
**dadurch gekennzeichnet,**
daß der Eintritts- und der Austrittsflansch (2, 4) mit Gehäusen (36) weiterer Wärmetauschermodule (35) korrespondierend und koppelbar ausgebildet sind.

14. Wärmeaustauscher nach einem der vorhergehenden Ansprüche,
**dadurch gekennzeichnet,**
daß das Gehäuse (7) mit tangential angeordneten Heizmediumstutzen (27, 28) ausgerüstet ist.

15. Wärmeaustauscher nach einem der vorhergehenden Ansprüche,
**dadurch gekennzeichnet,**
daß eingangs- und ausgangsseitig zwei um 180° versetzte Heizmediumstutzen (27 bzw. 28) vorgesehen sind.

16. Wärmeaustauscher nach einem der vorhergehenden Ansprüche,
**dadurch gekennzeichnet,**
daß die Lage der Rohre (18, 20) über im Abstand über die Länge des Gehäuses (7) verteilt angeordnete Halterungen (30) stabilisiert ist.

17. Wärmeaustauscher nach Anspruch 16,
**dadurch gekennzeichnet,**
daß die Halterungen (30) bei dem gewendelten Kapillarrohr (19) als sternförmige Träger (31) ausgebildet sind.

18. Wärmeaustauscher nach Anspruch 16,
**dadurch gekennzeichnet,**
daß die Halterungen (30) bei der mäanderförmigen Rohrschlange (21) als Distanzscheiben ausgebildet sind.

19. Wärmeaustauscher nach einem der vorhergehenden Ansprüche,
**dadurch gekennzeichnet,**
daß mehrere Wärmetauschermodule (34, 35) sternförmig parallel geschaltet sind.

20. Wärmeaustauscher nach einem der vorhergehenden Ansprüche,
**dadurch gekennzeichnet,**
daß drei Wärmetauschermodule (34, 35, 35') eine Kompletteinheit (37) mit Aufheiz-, Halte- und Kühlstufe (38, 39, 40) bildend miteinander verbunden sind.

21. Wärmeaustauscher nach Anspruch 20,
**dadurch gekennzeichnet,**
daß die mittlere Haltestufe (39) ohne Zwischenschaltung von Eintritts- bzw. Austrittsflansch (2, 4) direkt mit der Aufheiz- und der Kühlstufe (38, 40) verbunden ist.

22. Wärmetauscher nach einem der vorhergehenden Ansprüche,
**dadurch gekennzeichnet,**
daß die Zuteilungskanäle (15, 16) und/oder die Rohre (18, 20) eine Innenwandbeschichtung (46) aufweisen, die aus einem hydro- oder lyophoben Material bestehen.

23. Wärmetauscher nach einem der Ansprüche 1 - 21
**dadurch gekennzeichnet,**
daß die Zuteilungskanäle (15, 16) und/oder die Rohre (18, 20) insgesamt aus einem hydro- oder lyophoben Werkstoff bestehend ausgebildet sind.

24. Wärmetauscher nach einem der vorhergehenden Ansprüche,
**dadurch gekennzeichnet,**
daß die Zuteilungskanäle (15, 16) mit Verbindungsstutzen (47) versehen sind, die mit einem ein Trennmedium (42) aufweisenden und über ein Zuteilungssystem (50) verfügenden Behälter (48) verbunden sind.

25. Wärmetauscher nach Anspruch 24,
**dadurch gekennzeichnet,**
daß der Behälter (48) als Inertgas- oder Dampfbehälter ausgebildet ist.

26. Wärmetauscher nach einem der Ansprüche 24 oder 25
**dadurch gekennzeichnet,**
daß das Zuteilungsssystem (50) alle, einzelne oder Gruppen von Zuteilungskanälen (15, 16) oder Rohren (18, 20) eine segmentierende Flüssigkeitsströmung erzielend ausgelegt ist.

27. Verfahren zur thermischen Konditionierung von Stoffgemischen oder für die Sterilisation von Flüssigkeiten, die mit Mikroorganismen kontaminiert sind oder sein können, bei dem die Stoffgemische oder Flüssigkeiten indirekt erhitzt werden,
**dadurch gekennzeichnet,**
daß der Stoffgemisch- oder Flüssigkeitsstrom in kleine, genau definierte und ungefähr die gleiche Menge aufweisende Teilströme unterteilt und jeder Teilstrom mit gleicher Geschwindigkeit für einen vorgegebenen Zeitraum indirekt durch die Rohrwand gleichmäßig und schnell erhitzt und in die einzelnen Teilströme im vorgegebenen Abstand ein sich mit diesen nicht mischendes Trennmedium eingegeben wird.

28. Verfahren nach Anspruch 27,
**dadurch gekennzeichnet,**
daß die Teilströme in miteinander verbundenen Modulen getrennt aufgeheizt, auf Temperatur gehalten und anschließend gekühlt und dann erst wieder zusammengeführt werden.

29. Verfahren nach Anspruch 27,
**dadurch gekennzeichnet,**
daß die Teilströme zwischen Wänden mit einer Innenwandbeschichtung aus einem Grenzflächeneffekte unterbindenden Werkstoff, vorzugsweise aus Paraffinen oder ähnlichen Verbindungen hindurchgeführt werden.

30. Verfahren nach Anspruch 27,
**dadurch gekennzeichnet,**
daß das Trennmedium in einen, mehrere oder in Gruppen von Teilströmen eingeschleust wird.

31. Verfahren nach Anspruch 27,
**dadurch gekennzeichnet,**
daß als Trennmedium Inertgas- oder Dampfblasen verwendet werden.

## Claims

1. Heat exchanger (1) for the thermal conditioning of mixtures of substances or for the sterilisation of liquids, which are or may be contaminated by micro-organisms with an inlet and outlet flange (2, 4) having at least one connection opening for a central tube (12, 13) and a tube nest (3) connecting the latter, which is sealed from the environment by a housing (7) with a connection (27, 28) for the supply and removal of the heating medium, wherein the tube nest (3) comprises several tubes (18, 20) of equal length and cross section, each of the tubes (18, 20) is in connection with the respective central tube (12, 13) by separate metering channels (15, 16) of equal cross section and length, and the tubes are in the form of capillary tubes, the defined cross section and wall thickness of which permit an even and rapid increase in temperature.

2. Heat exchanger according to claim 1, **characterised in that** the tubes (18, 20) have a circular or oval cross section.

3. Heat exchanger according to claim 1, **characterised in that** the tubes (18, 20) have a rectangular cross section.

4. Heat exchanger according to one of the preceding claims, **characterised in that** the tubes (18, 20) have an inner diameter or an edge length of 0.5 to 5 mm, preferably 1.0 to 3.0 mm.

5. Heat exchanger according to one of the preceding claims, **characterised in that** the tubes (18, 20) have a wall thickness of 0.05 to 1 mm, preferably 0.1 to 0.3 mm.

6. Heat exchanger according to claim 1, **characterised in that** the tubes (18, 20) are distributed evenly over the cross section of the housing (7), and are designed to be bent or wound like a coil or winding.

7. Heat exchanger according to claim 1, **characterised in that** the inner and/or outer walls (24, 25) of the tubes (18, 20) are profiled.

8. Heat exchanger according to claim 1, **characterised in that** the metering channels (15, 16) are designed to be star shaped running from the central bore (14) connected with the central tube (12) to the tubes (18, 20) arranged at equal distances in a circle around the central axis (17).

9. Heat exchanger according to one of the preceding claims, **characterised in that** the metering channels (15, 16) are assigned individual or group acting control elements to vary the flow rate by pressurising different tubes (18, 20) designed as capillary tubes .

10. Heat exchanger according to one ofthe preceding claims, **characterised in that** the metering channels (15, 16) are designed to be integrated in the inlet flange (2) or outlet flange (4), and in that an associated front flange (5, 6) of the housing (7) has a continuous flat surface.

11. Heat exchanger according to claim 10, **characterised in that** the two front flanges (5, 6) are connected by support tubes (8) which receive connecting screws (9), and in the front flange (5, 6) and the inlet or outlet flange (2, 4) are connected by separate holding screws (10, 11).

12. Heat exchanger according to one of claims 10 or 11, **characterised in that** the two front flanges (5, 6) are connected directly with the housing (7) by soldering or welding.

13. Heat exchanger according to one of the preceding claims, **characterised in that** the inlet and outlet flange (2, 4) are designed to correspond and be coupled with the housings (36) of additional heat exchanger modules (35).

14. Heat exchanger according to one of the preceding claims, **characterised in that** the housing (7) is equipped with tangentially arranged heating medium connections (27, 28).

15. Heat exchanger according to one of the preceding claims, **characterised in that** on the input and output side two heating medium connections (27 or 28) are provided offset by 180°.

16. Heat exchanger according to one ofthe preceding claims,**characterised in that** the position of the tubes (18, 20) is stabilised by holders (30) arranged spaced apart over the length of the housing (7).

17. Heat exchanger according to claim 16, **characterised in that** the holders (30) with a coiled capillary tube (19) are designed as star-shaped bearers (31).

18. Heat exchanger according to claim 16, **characterised in that** the holders (30) with a winding tube (21) are designed as spacing discs.

19. Heat exchanger according to one of the preceding claims, **characterised in that** several heat exchanger modules (34, 35) are connected parallel to one another in a star shape.

20. Heat exchanger according to one of the preceding claims, **characterised in that** three heat exchanger modules (34, 35, 35') are connected together forming a complete unit (37) with a heating, holding and cooling stage (38, 39, 40).

21. Heat exchanger according to claim 20, **characterised in that** the middle holding stage (39) is connected directly without the interconnection of an inlet or outlet flange (2, 4) with the heating and the cooling stage (38. 40).

22. Heat exchanger according to one of the preceding claims, **characterised in that** the metering channels (15, 16) and/or the tubes (18, 20) have a inner wall coating (46) which is made of a hydrophobic or lyophobic material.

23. Heat exchanger according to one of claims 1 - 21 , **characterised in that** the metering channels (15, 16) and/or the tubes (18, 20) are designed as a whole to be made of a hydrophobic or lyophobic material.

24. Heat exchanger according to one of the preceding claims, **characterised in that** the metering channels (15, 16) are provided with connecting points (47) which are connected with a container (48) having a separating medium (42) and a metering system (50).

25. Heat exchanger according to claim 24, **characterised in that** the container (48) is designed as an inert gas or steam container.

26. Heat exchanger according to one of claims 24 or 25, **characterised in that** the metering system (50) all, individual or groups of metering channels (15, 16) or tubes (18, 20) is designed to achieve a segmenting liquid flow.

27. Method for the thermal conditioning of mixtures of substances or for the sterilisation of liquids, which are or may be contaminated by micro-organisms, in which the mixtures or liquids are heated indirectly, **characterised in that** the mixture or liquid flow is divided into small, precisely defined part flows in approximately the same amount, and each part flow at the same speed for a given time period is heated indirectly evenly and rapidly by the tube wall, and a separating medium that does not mix with the latter is added into the individual part flows at a given distance.

28. Method according to claim 27, **characterised in that** the part flows are heated separately in connected modules, kept to a temperature and afterwards cooled and only then combined again.

29. Method according to claim 27, **characterised in that** the part flows are guided through between walls with an inner wall coating made of a material preventing interface effects, preferably paraffin or similar compounds.

30. Method according to claim 27, **characterised in that** the separating medium is injected in one, several or in groups of part flows.

31. Method according to claim 27, characterised in that inert gas or steam bubbles are used as the separating medium.

## Revendications

1. Echangeur de chaleur (1) pour le conditionnement thermique de mélanges de substances ou pour la stérilisation de liquides qui sont ou peuvent être contaminés par des micro-organismes, comportant une bride d'entrée et une bride de sortie (2,4) qui possèdent au moins une ouverture de raccordement pour un tube central (12,13), et un faisceau de tubes (3) qui relie ces brides et qui est fermé vis-à-vis de l'environnement par un carter (7) comportant des tubulures (27,28) pour l'amenée et l'évacuation du fluide de chauffage, et dans lequel le faisceau de tubes (3) comporte plusieurs tubes (18,20) qui sont de même longueur et possèdent des sections transversales identiques, chacun des tubes (18,20) est relié par des canaux séparés de répartition (15,16), qui possèdent la même section transversale et la même longueur, au tube central respectif (12,13), et les tubes sont agencés sous la forme de tubes capillaires dont la section transversale et l'épaisseur de paroi définies permettent un accroissement uniforme et rapide de la température.

2. Echangeur de chaleur selon la revendication 1, caractérisé en ce que les tubes (18,20) possèdent une section transversale de forme circulaire ou ovale.

3. Echangeur de chaleur selon la revendication 1, caractérisé en ce que les tubes (18,20) possèdent une section transversale de forme rectangulaire.

4. Echangeur de chaleur selon l'une des revendications précédentes, caractérisé en ce que les tubes (18, 20) possèdent un diamètre intérieur et une longueur de bord comprise entre 0,5 et 5 mm et de préférence entre 1,0 et 3,0 mm.

5. Echangeur de chaleur selon l'une des revendications précédentes, caractérisé en ce que les tubes (18,20) possèdent une épaisseur de paroi comprise entre 0,05 et 1 mm, de préférence entre 0,1 et 0,3 mm.

6. Echangeur de chaleur selon la revendication 1, caractérisé en ce que les tubes (18,20) sont disposés en étant répartis uniformément sur la section transversale du carter (7) et sont coudés de manière à posséder une forme hélicoïdale ou sinueuse ou sont enroulés.

7. Echangeur de chaleur selon la revendication 1, caractérisé en ce que les parois intérieures et/ou les parois extérieures (24,25) des tubes (18,20) sont profilées.

8. Echangeur de chaleur selon la revendication 1, caractérisé en ce que les canaux de répartition (15,16) sont disposés en étoile de manière à s'étendre depuis le perçage central (14) relié au tube central (12), en direction des tubes (18,20) et sont disposés en étant régulièrement espacés selon une disposition circulaire autour de l'axe médian (17).

9. Echangeur de chaleur selon l'une des revendications précédentes, caractérisé en ce que des organes de régulation agissant individuellement ou par groupes pour modifier le débit en chargeant différents tubes (18,20) agencés sous la forme de tubes capillaires, sont associés aux canaux de répartition (15,16).

10. Echangeur de chaleur selon l'une des revendications précédentes, caractérisé en ce que les canaux de répartition (15,16) sont intégrés dans la bride d'entrée (2) et dans la bride de sortie (4) et en ce que la bride frontale associée (5,6) du carter (7) possède une surface complètement plane.

11. Echangeur de chaleur selon la revendication 10, caractérisé en ce que les deux brides frontales (5,6) sont reliées par l'intermédiaire de tubes de support (8) qui reçoivent des boulons de liaison (9) et en ce que les brides frontales (5,6) et la bride d'entrée et la bride de sortie (2,4) sont reliées par l'intermédiaire de boulons séparés de retenue (10,11).

12. Echangeur de chaleur selon l'une des revendications 10 ou 11, caractérisé en ce que les deux brides frontales (5,6) sont reliées directement au carter (7) par brasage ou par soudage.

13. Echangeur de chaleur selon l'une des revendications précédentes, caractérisé en ce que la bride d'entrée et la bride de sortie (2,4) sont réalisées de manière à correspondre aux carters (36) d'autres modules d'échangeurs de chaleur (35) et de manière à pouvoir y être couplées.

14. Echangeur de chaleur selon l'une des revendications précédentes, caractérisé en ce que le carter (7) est équipé de tubulures tangentielles (27,28) pour le fluide de chauffage.

15. Echangeur de chaleur selon l'une des revendications précédentes, caractérisé en ce que deux tubulures (27 et 28) pour le fluide de chauffage, qui sont décalées de 180°, sont prévues sur le côté entrée et sur le côté sortie.

16. Echangeur de chaleur selon une ou plusieurs des revendications précédentes, caractérisé en ce que la position des tubes (18,20) est stabilisée au moyen de dispositifs de retenue (30) distants les uns des autres en étant répartis sur la longueur du carter (7).

17. Echangeur de chaleur selon la revendication 16, caractérisé en ce que les dispositifs de retenue (30) sont agencés sous la forme d'un support en étoile (31) dans le cas du tube capillaire hélicoïdal (19).

18. Echangeur de chaleur selon la revendication 16, caractérisé en ce que les dispositifs de retenue (30) sont agencés sous la forme de disques entretoises dans le cas du serpentin tubulaire de forme sinueuse (21).

19. Echangeur de chaleur selon l'une des revendications précédentes, caractérisé en ce que plusieurs modules (34,35) d'échangeurs de chaleur sont branchés en parallèle selon une disposition en étoile.

20. Echangeur de chaleur selon l'une des revendications précédentes, caractérisé en ce que trois modules (34,35,35') d'échangeurs de chaleur sont reliés entre eux de manière à former une unité complète (37) comportant des étages de chauffage, de maintien et de refroidissement (38,39,40).

21. Echangeur de chaleur selon la revendication 20, caractérisé en ce que l'étage médian de maintien (39) est relié directement à l'étage de chauffage et l'étage de refroidissement (38,40), sans montage intercalé de brides d'entrée et de sortie (2,4).

22. Echangeur de chaleur selon l'une des revendications précédentes, caractérisé en ce que les canaux de répartition (15,16) et/ou les tubes (18,20) possèdent un revêtement de paroi intérieure (46), qui est réalisé en un matériau hydrophobe ou lyophobe.

23. Echangeur de chaleur selon l'une des revendications 1-21, caractérisé en ce que les canaux de répartition (15,16) et/ou les tubes (18,20) sont réalisés globalement en un matériau hydrophobe ou lyophobe.

24. Echangeur de chaleur selon l'une des revendications précédentes, caractérisé en ce que les canaux de répartition (15,16) sont équipés de tubulures de raccordement (47), qui sont reliées à un récipient (48) qui comporte un milieu de séparation (42) et est équipé d'un système de répartition (50).

25. Echangeur de chaleur selon la revendication 24, caractérisé en ce que le récipient (48) est agencé sous la forme d'un réservoir à gaz inerte ou à vapeur.

26. Echangeur de chaleur selon l'une des revendications 24 ou 25, caractérisé en ce que le système de répartition (50) est conçu de manière que la totalité, quelques-uns ou des groupes de canaux de répartition (15,16) ou de tubes (18,20) produisent un écoulement de liquide à segmentation.

27. Procédé pour le conditionnement thermique de mélanges de substances ou pour la stérilisation de liquides qui sont ou peuvent être contaminés par des micro-organismes, selon lequel on chauffe de façon indirecte les mélanges de substances ou les liquides, caractérisé en ce que le courant du mélange de substances ou du liquide est subdivisé en de petits courants partiels définis de façon précise et contenant approximativement la même quantité, en ce que chaque courant partiel est chauffé uniformément et rapidement avec la même vitesse pendant un intervalle de temps prédéterminé, indirectement par la paroi du tube, et en ce que dans les courants partiels individuels est introduit, à un intervalle prédéterminé, un milieu de séparation qui ne se mélange pas à ces courants partiels.

28. Procédé selon la revendication 27, caractérisé en ce que les courants partiels sont chauffés, maintenus à température et ensuite refroidis séparément dans des modules reliés entre eux et sont ensuite à nouveau réunis.

29. Procédé selon la revendication 27, caractérisé en ce que les courants partiels sont guidés entre des parois possédant un revêtement intérieur formé d'un matériau empêchant des effets de surface limite, de préférence des paraffines ou des composés similaires.

30. Procédé selon la revendication 27, caractérisé en ce que le milieu de séparation est introduit dans un courant partiel, dans plusieurs courants partiels ou dans des groupes de courants partiels.

31. Procédé selon la revendication 27, caractérisé en ce qu'on utilise, comme milieu de séparation, des bulles de gaz inerte ou de vapeur.
